Europäisches Patentamt

European Patent Office

Office européen des brevets

(19)

(11) Veröffentlichungsnummer : **0 186 090**
**B1**

(12) # EUROPÄISCHE PATENTSCHRIFT

(45) Veröffentlichungstag der Patentschrift :
30.08.89

(51) Int. Cl.⁴ : **A 61 K 31/375**, A 61 K 9/16

(21) Anmeldenummer : 85116039.0

(22) Anmeldetag : 16.12.85

(54) **Verfahren zur Herstellung von Ascorbinsäure-Granulat.**

(30) Priorität : 24.12.84 DE 3447422

(43) Veröffentlichungstag der Anmeldung :
02.07.86 Patentblatt 86/27

(45) Bekanntmachung des Hinweises auf die Patenterteilung : 30.08.89 Patentblatt 89/35

(84) Benannte Vertragsstaaten :
CH DE FR GB IT LI NL

(56) Entgegenhaltungen :
EP–A– 0 049 143
FR–A– 2 150 903
FR–A– 2 380 777
GB–A– 844 772
US–A– 2 820 741

(73) Patentinhaber : BASF Aktiengesellschaft
Carl-Bosch-Strasse 38
D-6700 Ludwigshafen (DE)

(72) Erfinder : Buehler, Volker, Dr.
In den Weingaerten 14
D-6706 Wachenheim (DE)
Erfinder : Hofmann, Friedbert, Dr.
Rehbachstrasse 34a
D-6708 Neuhofen (DE)
Erfinder : Heinz, Robert
Heilbronner Strasse 2
D-6800 Mannheim 51 (DE)

EP 0 186 090 B1

## Beschreibung

Die Erfindung betrifft ein Verfahren zur Herstellung von Ascorbinsäure-Granulat zur Direkttablettierung mit Polyvinylpyrrolidon (PVP) als Bindemittel nach dem Kompaktierverfahren. Aus diesem Granulat lassen sich stabile Tabletten herstellen.

Das Verpressen von Ascorbinsäure in höherer Konzentration zu Tabletten erfordert eine vorherige Granulation mit einem Bindemittel. Als eines der Bindemittel mit höchster Bindekraft, das gleichzeitig den aus dem Granulat hergestellten Tabletten eine hohe Zerfallsgeschwindigkeit vermittelt, kommt PVP in Betracht. Es hat den weiteren Vorteil, daß es wasserlöslich ist, was z. B. für den Einsatz in Brausetabletten wichtig ist. Es ist jedoch bekannt, daß mit PVP granulierte Ascorbinsäure zu instabilen Tabletten führt, und daß Wasser die Stabilität stark herabsetzt (Peter C. Schmidt, Deutsche Apotheker Zeitung 122 (1982), Nr. 3, S. 111, Abb. 12 und S. 110, rechte Spalte oben). Daher wird empfohlen, mit wasserfreien Lösungsmitteln zu granulieren (S.P. Gladkikh, Khimiko-Farmatsevticheskii Zhurnal, Vol. 4, No. 12, Seiten 37 bis 42, Dezember 1970, engl. Übersetzung 1971, Consultants Büro, A Division of Plenum Publishing Corporation, 227 West 17th Street, New York, N.Y. 10011, Seite 702, Absatz 6). Besonders störend ist die Tatsache, daß die Tabletten noch vor einem merklichen Aktivitätsverlust zu einer Verfärbung ins Gelbliche bis Bräunliche neigen. S. Lee et al. (J. Pharm. Sci. 54 (1965), Nr. 8, S. 1156, Tab. VI) haben gezeigt, daß mit PVP als Bindemittel selbst die Verwendung von wasserfreien Lösungsmitteln nicht zu stabilen Granulaten führt. Außerdem wird die Verwendung von organischen Lösungsmitteln heutzutage aus Umweltschutzgründen nach Möglichkeit vermieden.

In handelsüblichen Granulaten von Ascorbinsäure zur Direkt-Tablettierung werden andere Bindemittel, nämlich Stärke und Ethylcellulose verwendet. Auch mit diesen werden nicht immer farbstabile Granulate erzielt, und vor allem die Zerfallseigenschaften und die Wasserlöslichkeit der Tabletten sind schlechter bei Verwendung dieser Bindemittel im Vergleich zu PVP.

Der Erfindung lag daher die Aufgabe zugrunde, ein Verfahren zur Herstellung von farbstabilen Ascorbinsäure-Granulaten mit PVP als Bindemittel zur Direkt-Tablettierung zu entwickeln. Mit Farbstabilität ist hier gemeint, daß das Granulat bei verschlossener Lagerung nach mindestens einem Jahr bei Raumtemperatur oder nach 3 Monaten bei 40 °C keine oder fast keine Verfärbung zeigt. « Verschlossene Lagerung » bedeutet Luftabschluß, nicht Luftausschluß. « Direkttablettierung » besagt, daß das Material mit oder ohne Abmischen mit anderen Hilfsstoffen ohne Granulation zu Tabletten verpreßt werden kann.

Die Aufgabe wird gelöst durch ein Verfahren zur Herstellung von Ascorbinsäure-Granulat zur Direkttablettierung nach dem Kompaktierverfahren, dadurch gekennzeichnet, daß man die trockene Ascorbinsäure (Wassergehalt unter 0.3 %) mit einer Korngröße im Bereich von 30 bis 500, vorzugsweise 50 bis 250 $\mu$m, im Gemisch mit 0,8 bis 8, vorzugsweise 1 bis 6 Gew. %, bezogen auf das fertige Granulat, an löslichem PVP mit einem Wassergehalt von 0,3 bis 5, vorzugsweise 0,5 bis 4 Gew. %, bezogen auf das PVP, in an sich üblicher Weise kompaktiert und zerkleinert. Maximal die Hälfte des löslichen PVP kann durch feinteiliges, vorzugsweise mikronisiertes unlösliches PVP, dessen Wassergehalt im gleichen Bereich liegt, ersetzt werden.

Beim Kompaktieren wird die Mischung von Wirkstoff (hier : Ascorbinsäure) und Bindemittel und gegebenenfalls weiteren Hilfsstoffen zwischen zwei Walzen zu einem Strang verpreßt und anschließend zerkleinert, wobei (zumindest im Verfahren der vorliegenden Erfindung) die Fraktion mit Teilchendurchmessern von 100 bis 1 000, vorzugsweise 200 bis 800 $\mu$m zur Herstellung von Tabletten verwendet und die feineren und gröberen Anteile noch einmal im Kompaktierverfahren eingesetzt werden.

Der Zusatz von feinteiligem (Teilchengröße zu 100 Gew. % unter 100 $\mu$m), vorzugsweise mikronisiertem (Teilchengröße zu 100 Gew. % unter 50, zu 90 Gew. % unter 10 $\mu$m) unlöslichem (durch Popcorn-Polymerisation hergestelltem, also überwiegend « physikalisch vernetztem ») PVP hat sich hinsichtlich der Farbstabilität besonders bewährt, hat aber den (nur bei Brausetabletten zum Tragen kommenden) Nachteil, daß das Produkt nicht mehr klar wasserlöslich ist. Das lösliche PVP hat einen K-Wert nach der Britischen Pharmakopöe 1980, Addendum 1982, S. 93, im Bereich von 20 bis 95, vorzugsweise 28 bis 95.

Beim Verpressen des Granulats zu Tabletten können die üblichen Hilfsmittel mitverwendet werden, beispielsweise zusätzliche Bindemittel wie Stärke, Cellulose, Dextrin, Amylopectin, alle gegebenenfalls chemisch modifiziert, Gelatine, Traganth ; Füllstoffe wie Calciumsulfat, Amylose, Lactose, Cellulose ; Wein- oder Zitronensäure und Natriumhydrogencarbonat für Brausetabletten ; Gleitmittel wie Talkum, Magnesium- oder Calciumstearat, gegebenenfalls im Gemisch mit Maisstärke, wachsartige Substanzen, z. B. gesättigte Fettsäuren oder ein hydriertes Glycerin in Mischung mit einem Stearat und/oder Titandioxid, Polyethylenglykol, Silicagel, Calciumarachinat ; ferner Geschmackstoffe wie Saccharin, Cyclamat, Zucker oder Aromastoffe wie Vanillin oder Orangeextrakt, sowie Farbstoffe und Antioxidantien. Die Tabletten können auch einen Überzug erhalten.

Das erfindungsgemäß hergestellte Ascorbinsäure-Granulat zur Direkttablettierung ist entsprechenden handelsüblichen Produkten hinsichtlich der Farbstabilität des Granulats selbst und der daraus hergestellten Tabletten z.T. weit überlegen. Außerdem ist die Zerfallszeit in künstlichem Magensaft von Tabletten, die aus dem erfindungsgemäßen Granulat nach üblichen Formulierungen hergestellt wurden, bedeutend kürzer als bei Tabletten, die in gleicher Weise aus handelsüblichen Granulaten hergestellt wurden. Schließlich ist bei Verzicht auf den Einsatz von unlöslichem PVP das erfindungsgemäße Granulat im Gegensatz zu handelsüblichem zur Herstellung von klarlöslichen Brausetabletten geeignet, wobei die vorgenannten Vorteile weitgehend erhalten bleiben.

Die in den Beispielen genannten Teile beziehen sich auf das Gewicht.

Beispiel 1

Herstellung

| | |
|---|---|
| Ascorbinsäure (max. 10 Gew. % größer als 150 $\mu$m, mind. 65 Gew.% größer als 50 $\mu$m) | 96 kg |
| lösliches Polyvinylpyrrolidon (K-Wert 28-32, Wassergehalt 1,6 %) | 4 kg |

Die homogene Mischung wurde trocken kompaktiert und zerkleinert. Der Grobanteil über 1 000 $\mu$m wurde abgesiebt.

Stabilität

Das so hergestellte Kompaktat zeigte im Gegensatz zu den handelsüblichen Ascorbinsäuregranulaten für die Direkttablettierung nach Lagerung unter Luftabschluß bei 40 °C nach 3 Monaten keinerlei Veränderung der Farbe. Auch der Gehalt der Ascorbinsäure veränderte sich nicht.

Anwendung

In der folgenden Brausetablettenformulierung wurde das Kompaktat mit den handelsüblichen Granulaten mit Hilfe der Direkttablettierung verglichen :

| | |
|---|---|
| Ascorbinsäure-Kompaktat bzw. Granulat | 1 000 mg |
| Weinsäure (Pulver) | 200 mg |
| Sorbit | 200 mg |
| Natriumhydrogencarbonat | 172 mg |
| Polyethylenglykol vom mittleren Molgewicht 6 000 | 60 mg |

Die Brausetabletten (20 mm Durchmesser, biplanar, mit 50 kN Preßdruck hergestellt) zeigten folgende Eigenschaften :

| Produkt | Harte | Zerfall in Wasser | Abrieb* |
|---|---|---|---|
| Kompaktat (Beispiel 1) | 140 N | 2 - 3 min | 0,1 % |
| handelsübliche Granulate | 150 N | 9 min | 0,2 % |

\* Friabilator nach Hoffman-LaRoche

Die aus dem Kompaktat hergestellten Brausetabletten waren farbstabiler als die meisten Vergleichspräparate und lösten sich klar in Wasser im Gegensatz zu den aus den handelsüblichen Granulaten hergestellten Brausetabletten.

Beispiel 2

Herstellung

| | |
|---|---|
| Ascorbinsäure (max. 10 Gew. % größer als 150 $\mu$m. mind. 65 Gew. % größer als 50 $\mu$m) | 96 kg |
| lösliches Polyvinylpyrrolidon (K-Wert 30, Wassergehalt 1,6 %) | 3 kg |
| unlösliches Polyvinylpyrrolidon, mikronisiert (Wassergehalt 3,5 %) | 1 kg |

Die Verarbeitung erfolgte wie bei Beispiel 1.

Stabilität

Das so hergestellte Kompaktat hatte die gleiche Stabilität wie das Kompaktat gemäß Beispiel 1.

Anwendung

In der folgenden Tablettenformulierung wurde das Kompaktat mit den handelsüblichen Granulaten verglichen :

| | |
|---|---|
| Ascorbinsäure-Kompaktat bzw. Granulat | 300 mg |
| Mikrokristalline Cellulose | 200 mg |
| Polyethylenglykol 6 000 | 20 mg |
| Talkum + Silicagel + Calciumarachinat (8 + 1 + 1) | 5 mg |

Die Tabletten (12 mm Durchmesser, biplan facettiert, mit 10 kN Preßdruck hergestellt) hatten folgende Eigenschaften :

| Ausgangsmaterial | Härte | Zerfallszeit in künstl. Magensaft | Abrieb* |
|---|---|---|---|
| erf.gem. Kompaktat | 115 N | 90 sec | < 0.1 % |
| handelsübliche Granulate | 130-150 N | 19~20 min | < 0.1 % |

\* Friabilator nach Hoffman-LaRoche

## Beispiel 3

| | |
|---|---|
| Ascorbinsäure (mind. 65 Gew. % größer als 150 µm. max. 5 Gew. % größer als 500 µm) | 98 kg |
| lösliches Polyvinylpyrrolidon (K-Wert 90, Wassergehalt 3,1 %) | 1 kg |
| unlösliches Polyvinylpyrrolidon, mikronisiert (Wassergehalt 3,5 %) | 1 kg |

Die Verarbeitung erfolgte wie bei Beispiel 1.

## Patentansprüche

1. Verfahren zur Herstellung von Ascorbinsäure-Granulat zur Direkttablettierung nach dem Kompaktierverfahren, dadurch gekennzeichnet, daß man die Ascorbinsäure in Form von Kristallen oder Kristallbruchstücken, von denen höchstens 40 Gew. % einen größten Teilchendurchmesser unter 50 µm und höchstens 15 Gew. % über 500 µm besitzen, im Gemisch mit 0,8 bis 8 Gew. % an löslichem Polyvinylpyrrolidon mit einem Wassergehalt von 0,3 bis 5 % in an sich üblicher Weise kompaktiert, indem man die Mischung, die auch weitere übliche Hilfsstoffe enthalten kann, zwischen zwei Walzen zu einem Strang verpreßt und anschließend zerkleinert.

2. Verfahren zur Herstellung von Ascorbinsäure-Granulat nach Anspruch 1, dadurch gekennzeichnet, daß bis zur Hälfte des löslichen Polyvinylpyrrolidons durch unlösliches Polyvinylpyrrolidon ersetzt wird.

## Claims

1. A process for the preparation. of ascorbic acid granules for direct tableting by the compacting method, wherein the ascorbic acid in the form of crystals or crystal fragments, of which not more than 40 % by weight have a longest particle diameter of less than 50 µm and not more than 15 % by weight have a longest particle diameter greater than 500 µm, and as a mixture with from 0.8 to 8 % by weight of soluble polyvinylpyrrolidone having a water content of from 0.3 to 5 % is compacted in a conventional manner by pressing the mixture, which may also contain further conventional auxiliaries, between two rollers to give a strand, and comminuting the latter.

2. A process for the preparation of ascorbic acid granules as claimed in claim 1, wherein as much as half the soluble polyvinylpyrrolidone is replaced with insoluble polyvinylpyrrolidone.

## Revendications

1. Procédé de préparation de granulés d'acide ascorbique pour le pastillage direct selon le procédé par compactage, caractérisé par le fait que l'on compacte, de manière connue en soi, l'acide ascorbique sous forme de cristaux ou fragments de cristaux dont au plus 40 % en poids possèdent un diamètre de particules maximal inférieur à 50 µm et au plus 15 % en poids supérieur à 500 µm, en mélange avec 0,8 à 8 % en poids de polyvinylpyrrolidone soluble, avec une teneur en eau de 0,3 à 5 %, le mélange, qui peut contenir aussi d'autres auxiliaires usuels, étant transformé en boudin par pressage entre deux cylindres, puis concassé.

2. Procédé de préparation de granulés d'acide ascorbique selon la revendication 1, caractérisé par le fait que l'on remplace jusqu'à la moitié de la polyvinylpyrrolidone soluble par de la polyvinylpyrrolidone insoluble.